# EUROPEAN PATENT SPECIFICATION

(11) **EP 0 616 805 B1**
(45) Date of publication and mention of the grant of the patent: **10.06.1998**
(21) Application number: 94830122.1
(22) Date of filing: 21.03.1994
(51) Int. Cl.: A61K 31/22, A61K 31/205

(54) **Use of acyl L-carnitine gamma-hydroxybutyrate for the treatment of alcoholism**
Verwendung von Ester von Acyl-L-Carnitin mit Gammahydroxybuttersäure zur Behandlung von Alkoholismus
Utilisation d'acyl-L-carnitine gammahydroxybutyrate dans le traitement de l'alcoolisme

(30) Priority: 26.03.1993 IT RM930191
(43) Date of publication of application: 28.09.1994
(73) Proprietor: Sigma-Tau Industrie Farmaceutiche Riunite S.p.A., 00144 Roma (IT)
(72) Inventor: Scapagnini, Giovanni, I-90500 Catania (IT); Cavazza, Claudio, I-00186 Rome (IT)
(74) Representative: Fassi, Aldo, Dr.

(56) References cited:
- EP-A- 0 344 704
- EP-A- 0 442 850
- EP-A- 0 514 357
- NEUROPHARMACOLOGY vol. 23, no. 2B , 1984 pages 269 - 271 R. CORBETT ET AL. 'EFFECTS OF CARNITINE ON CHANGES CAUSED BY CHRONIC ADMINISTRATION OF ALCOHOL'
- THE BRITISH JOURNAL OF EXPERIMENTAL PATHOLOGY vol. 58, no. 6 , 1977 pages 606 - 615 C. ABU MURAD ET AL. 'HEPATIC TRIGLYCERIDE ACCUMULATION AND THE ETHANOL PHYSICAL WITHDRAWAL SYNDROME IN MICE'
- INTERNATIONAL JOURNAL OF CLINICAL PHARMACOLOGY RESEARCH vol. X, no. 1-2 , 1990 pages 101 - 107 E. TEMPESTA ET AL. 'ROLE OF ACETYL-CARNITINE IN THE TREATMENT OF COGNITIVE DEFICIT IN CHRONIC ALCOHOLISM'

## Description

The present invention relates to a new therapeutical use of acyl L-carnitine esters with gamma-hydroxybutyric acid of formula (I) 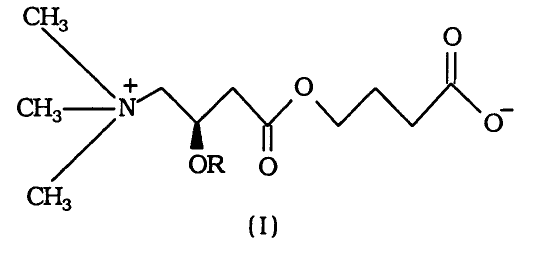 which represents them in the form of inner salts, wherein R is straight or branched acyl with from 2 to 5 carbon atoms, preferably acetyl, propionyl, n-butyryl, isobutyryl and isovaleryl,
or formula (I') 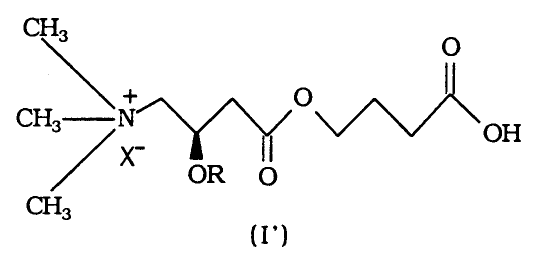 which represents them in the form of salts with pharmacologically acceptable acids, where R has the meaning indicated above and X⁻ is the anion of such an acid.

X⁻ is, for example: chloride; bromide; iodide, aspartate, particularly acid aspartate; citrate, particularly acid citrate; tartrate; phosphate, particularly acid phosphate: fumarate, particularly acid fumarate; glycerophosphate; glucose phosphate; lactate; maleate, particularly acid maleate; orotate; oxalate, particularly acid oxalate; sulphate, particularly acid sulphate; trichloroacetate; trifluoro-acetate and methanesulphonate.

The formula (I) or formula (I') esters are used for the preparation of a drug for the treatment of subjects abusing alcoholic beverages. In alcoholics, this drug suppresses withdrawal symptoms (such as tremors, perspiration, hyperreflexia, nausea, anxiety and convulsions) and the craving for alcohol.

The formula (I) and (I') esters are known compounds. In European patent application n° 0442 850 A1 (published on 21.08.91) their synthesis, physico-chemical characteristics and pharmacological activity in inhibiting neuronal degeneration (as occurring typically in Alzheimer's dementia and in Parkinson's disease) and in the treatment of coma are described.

In European patent application n° 0514 357 A1 (published on 19.11.92) a further therapeutic use of these formula (I) and (I') esters to produce a drug for the treatment of liver disease is described.

There is no relationship between these known uses and the use envisaged in this invention.

All the drugs used to date for the treatment of alcoholism present substantial drawbacks.

A number of the drugs commonly used in the treatment of alcohol withdrawal syndrome are similar to this with regard to their pharmacological effects. In fact, the most useful of those currently used are those with which alcohol develops a cross tolerance. All patients treated for withdrawal syndromes are potential candidates for SNC depressants though not all of them need them.

Paraldehyde, which was once extensively used in therapy, was completely abandoned on account of its disagreeable odor and a series of sudden, inexplicable deaths following its use.

Rarely used today are the fast-acting barbiturate (pentobarbital and secobarbital).

The drugs of choice are benzodiazepines such as chlordiazepoxide and diazepam. One serious drawback they present, however, is the fact that alcoholics taking chlordiazepoxide or diazepam may become intoxicated and even develop physical addiction and withdrawal syndrome.

The phenothiazines are not recommended since they do not control severe delirium tremens and also lower the threshold for attacks of epilepsy.

Lastly, a matter of some controversy is the therapeutic use of disulfiram which interferes with the metabolism of acetaldehyde (an intermediate product of the oxidation of alcohol) and produces an accumulation of it, thus causing toxic symptoms and severe discomfort. Drinking alcohol within 12 h of taking disulfiram produces facial flushing within 5-15 minutes, followed by intense vasodilatation of the face and neck with clouding of the conjunctiva, throbbing headache, tachycardia, hyperpnoea and perspiration. Within 30-60 minutes nausea and vomiting appear and can be so intense as to lead to hypertension, dizziness and sometimes fainting or collapse. The reaction lasts from one to three hours. The sense of malaise is so intense that few patients will risk drinking alcohol while taking disulfiram. Occasionally, this drug has also caused convulsions, cardiac arrhythmias and myocardial infarction.

It has now been found that formula (I) and formula (I') esters administered orally or parenterally at non-hypnotic doses suppress withdrawal symptoms and craving in alcoholics without causing the above-mentioned adverse or toxic reactions.

Reported here below are details of a number of in-vivo pharmacological studies which demonstrate the activity of one of the compounds as per the invention, namely isovaleryl L-carnitine gamma-hydroxybutyrate.

In the description following here below, VCGB stands for isovaleryl L-carnitine gamma-hydroxybutyrate, GHB for gamma-hydroxybutyric acid and IVC stands for isovaleryl L-carnitine.

### Sedative action of VCGB in alcohol-dependent rats as measured according to the Vogel test.

Wistar male rats (housed in groups of 5 per cage) were used, kept in conditions of constant light-dark alternation at 21°C and fed with standard laboratory feed. The animals were chronically administered 10% ethanol ad lib. for 6 months.

To assess the activity, if any, of VCGB on the anxiety component involved in the compulsive act of searching for alcohol, a modified version of the Vogel test was used as described by Keppler D. et al. in Exp. Mol. Path. 9, 279 (1968).

Prior to the start of the experiment the animals were deprived of water for 48 h. At the time of ethanol treatment suspension, the animals were divided into 4 groups who were given the following intraperitoneal administrations: the first group (A) received saline solution (15 ml/kg), the second group (B) VCGB (100 mg/kg), the third group (C) GHB (100 mg/kg) and the fourth group (D) IVC (100 ml/kg).

On the day of the test, the animals were provided with two drinking vessels, one containing water and the other a mixture of water and ethanol (90:10 v/v) in bottles, the metal spouts of which were connected up to a source of electricity: after every 5 licks at the drinking vessel an electric shock (1 mA) was delivered for 10 min. which obliged the animal to withdraw, overcoming its desire to drink the water-alcohol solution. Refusal of the animals to drink the water-alcohol solution, confining their attention only to the vessel containing water alone, was taken as an indication of efficacy. In evaluating the results, the numbers of licks were compared for treated animals versus controls over the 10 min. exposure period. The results are given here below in Table 1.

**TABLE 1**

| **Number of licks of treated animals over 10 min. exposure period.** | |
|---|---|
| Animal group | Number of licks |
| A (saline) | 38.5 ± 8.5 |
| B (VCGB) | 5.6 ± 1.14* |
| C (GHB) | 11 ± 2.4* ▲ |
| D (IVC) | 35.2 ± 7.4 |

| | |
|---|---|
| * P < 0.01 vs A | |
| ▲ P < 0.05 vs B | |

### Effects of VCGB on withdrawal syndrome in rats as measured by the Hunt-Majchrowicz method.

The experiment was performed in Wistar male rats housed in groups of 5 per cage under conditions of constant light-dark alternation at 21°C and fed with standard laboratory feed. The animals were chronically administered 10% ethanol ad lib. for 6 months.

Eight hours after suspension of ethanol administration, the animals were divided into 4 groups of 5 rats each.

The first group (A) was administered saline solution intraperitoneally (15 ml/kg), the second group (B) was given VCGB (100 mg/kg), again intraperitoneally, the third group (C) received GHB (100 mg/kg), while the fourth group (D) was given IVC (100 mg/kg). For the purposes of assessing the effects induced by the above-mentioned treatments on occurrence of alcohol withdrawal syndrome, tremors were measured in the rat, where, as in man, they are the most characteristic sign of withdrawal syndrome. Evaluation of the number of tremors was done using the method described by Hunt and Majchrowicz (see Hunt W.A. and Majchrovicz E., Journal of Pharmacology and Experimental Therapeutics, 213, 9-12 (1980). The results are given here below in Table 2.

**TABLE 2**

| Animal group | 30 min | 60 min | 90 min | 120 min |
|---|---|---|---|---|
| A (saline) | 6 ± 2.5 | 8 ± 1.5 | 7 ± 1.5 | 8 ± 2.5 |
| B (VCGB) | 0 ± 0.25 | 0 ± 0 | 0 ± 0.75 | 0 ± 0.25 |
| C (GHB) | 2 ± 0.5 | 1 ± 0.5 | 2 ± 1 | 3 ± 1 |
| D (IVC) | 4 ± 2 | 5 ± 2 | 5 ± 1.5 | 6 ±2 |

### Effects of VCGB on withdrawal syndrome as assessed by observing behavior of rats chronically treated with alcohol when subjected to sound stimulation.

The experiment was performed in Wistar male rats housed in groups of 5 per cage under constant light-dark alternation at 21°C and fed with standard laboratory feed. The animals were chronically administered 10% ethanol ad lib. for 6 months.

Eight hours after suspension of ethanol administration the animals were divided into 4 groups of 10 rats each. The first group (A) were administered saline solution (15 ml/kg) intraperitoneally, the second group (B) VCGB (100 mg/kg), again intraperitoneally, the third group (C) GHB (100 mg/kg) and the last group (D) IVC (100 mg/kg). For the purposes of evaluating the effects of the treatment on occurrence of alcohol withdrawal syndrome, the rats' susceptibility to onset of behavioral reactions (convulsions) due to emission of sound for 1 min by an electric bell (100 dB) was observed. The experiment was performed in all groups of rats one hour after treatment, and the results are given here below in Table 3.

**TABLE 3**

| Animal group | Animals with convulsions/animals treated |
|---|---|
| A (saline) | 9/10 |
| B (VCGB) | 1/10 |
| C (GHB) | 4/10 |
| D (IVC) | 6/10 |

### Effects of VCGB on lipoperoxidation in the CNS of rats receiving chronic ethanol treatment.

This study was conducted in order to assess what variations in malonaldehyde (MDA) occur following VCGB administration, MDA being a reliable and quantifiable index of the vulnerability of the central nervous system to damage due to free radicals as a result of the detrimental effects of ethanol.

The experiment was conducted in Wistar male rats housed in groups of 5 per cage under constant light-dark alternation at 21°C and fed with standard laboratory feed. Two groups of rats, each comprising 5 animals, were administered 10% ethanol in water throughout the treatment period. A third group of animals was kept on a standard laboratory diet without receiving any treatment. After two months of ethanol treatment the animals in the first group were administered VCGB in aqueous solution (100 mg/kg) in a single intraperitoneal dose. A few days after treatment, the animals were sacrificed and the brains promptly removed. MDA was measured using a modified micromethod described by Slater and Sawyer (Slater T. F. and Sawyer B. C., 1971, J. Biochem. Tokyo 8: 2180). The tissue was held for 10 min. at 0°C in Tris-HCl 0.05 M buffer at pH 7.4 and then homogenized. An aliquot (0.5 ml) of cerebral homogenate was extracted with 20% trichloroacetic acid (w/v). After centrifuging, 0.9 ml of supernatant were added to 1 ml of 0.67% thiobarbituric acid in Tris-HCl 0.026 M buffer at pH 7.0. The samples were placed in boiling water for 10 min, and after cooling absorbance was determined at 532 nm using a spectrophotometer. MDA was expressed in nmol/mg protein. Proteins were measured by the Smith method (Smith et al., 1985, Analyt. Biochem. 27: 502), using bicinchoninic acid as reagent. Table 4 here below shows the amount of MDA in control animals, in animals after administration of ethanol and in animals after administration of ethanol + VCGB.

**TABLE 4**

| **Distribution of cerebral MDA in control animals and after administration of ethanol and ethanol plus VCGB.** | |
|---|---|
| Animal group | MDA nmol/mg protein |
| Control | 0.543 ± 0.19 |
| Ethanol | 1.12 ± 0.13 |
| Ethanol + VCGB | 0.497 ± 0.15 |

The compounds of the present invention are administered orally or parenterally in any of the usual pharmaceutical forms which are prepared by conventional procedures well-known to the experts in these techniques. These forms such as tablets, capsules, solutions, syrups and the like, and injectable forms include both solid and liquid oral unitary dosage forms such as, for example, sterile solutions for vials and ampoules.

For these pharmaceutical forms the usual solvents, diluents and excipients are used. Optionally, preservative, sweetening and flavoring agents can be added. Non-exclusive examples of such substances are sodium carboxy-methylcellulose, polysorbate, sorbitol, starch, avicel®, talc and others which are evident to experts in pharmaceutical technology.

The dose administered will be decided by the primary care physician, bearing in mind the patient's age, body weight and general condition, on the basis of an appropriate professional assessment. Though effective results can be noted even at daily doses of from 5 to 8 mg/kg body weight, doses ranging from 10 to 50 mg/kg body weight are optimal. When considered necessary, larger doses can be given, owing to the low toxicity of the compounds covered by the invention.

The dosages envisaged, according to the pharmaceutical administration form, are, though not exclusively, the following:
- ampoules: : from 5 to 500 mg
- capsules: : from 15 to 50 mg
- tablets: : from 15 to 500 mg
- oral solutions: : from 15 to 50 mg

## Claims

1. Use of an ester of the formula (I) 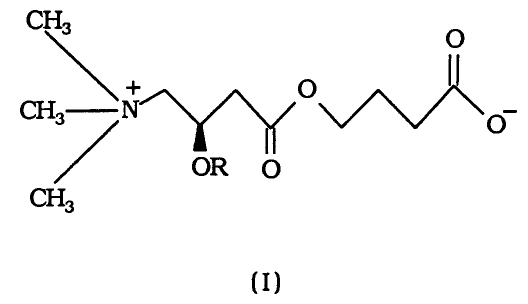 wherein R is straight or branched acyl with from 2 to 5 carbon atoms, or formula (I') 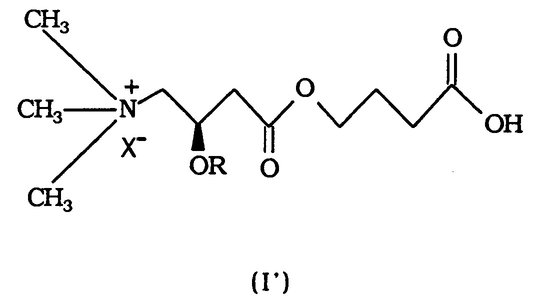 wherein R has the meaning indicated above and X⁻ is the anion of a pharmacologically acceptable acid, for the preparation of a drug to suppress withdrawal symptoms and craving for alcohol in alcoholics.

2. Use according to claim 1 above wherein R is chosen from among acetyl, propionyl, n-butyryl, isobutyryl and isovaleryl.

3. Use according to claims 1 or 2 above, in which X⁻ is chosen from among chloride; bromide, iodide; aspartate, particularly acid aspartate; citrate, particularly acid citrate; tartrate; phosphate, particularly acid phosphate; fumarate, particularly acid fumarate; glycerophosphate; glucose phosphate; lactate; maleate; particularly acid maleate; orotate; oxalate, particularly acid oxalate; sulphate, particularly acid sulphate; trichloroacetate; trifluoroacetate and methanesulphonate.

## Patentansprüche

1. Verwendung eines Esters der Formel (I). 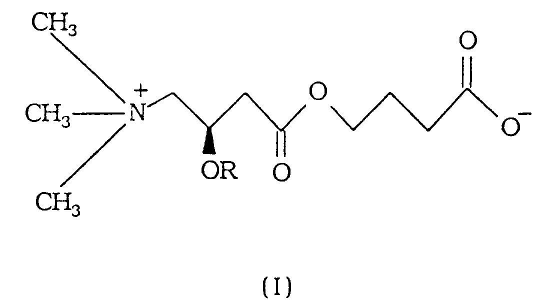 worin R ein geradkettiges oder verzweigtes Acyl mit 2 bis 5 Kohlenstoffatomen ist, oder der Formel (I'): 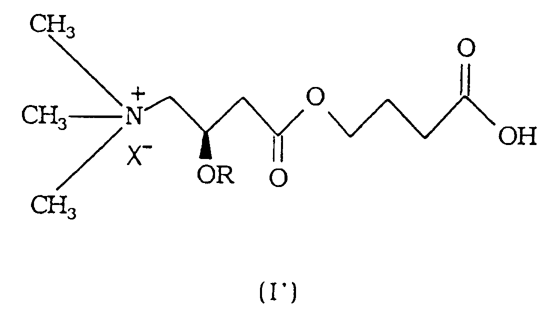 worin R die oben angegebene Bedeutung hat und X⁻ das Anion einer pharmakologisch akzeptablen Säure ist, zur Herstellung eines Arzneimittels, zum Unterdrücken von Entzugssymptomen und der Alkoholsucht bei Alkoholikern.

2. Verwendung nach Anspruch, worin R ausgewählt ist aus Acetyl, Propinyl, n-Butyryl, Isobutyryl und Isolvaleryl.

3. Verwendung nach den Ansprüchen 1 oder 2, worin X⁻ausgewählt ist aus Chlorid, Bromid, Jodid, Aspartat, insbesondere saures Aspartat, Citrat, insbesondere saures Citrat, Tartrat, Phosphat, insbesondere saures Phosphat, Fumarat, insbesondere saures Fumarat, Glycerophosphat, Glucosephosphat, Lactat, Maleat, insbesondere saures Maleat, Orotat, Oxalat, insbesondere saures Oxalat, Sulfat, insbesondere saures Sulfat, Trichloracetat, Trifluoracetat und Metansulfonat.

## Revendications

1. Utilisation d'un ester de formule (I) 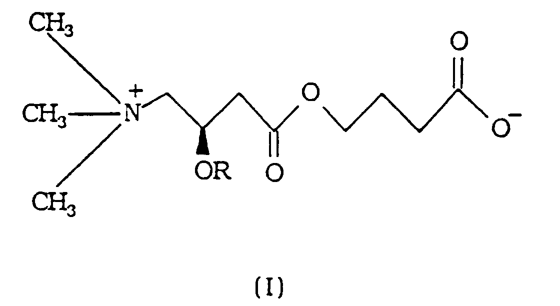 dans laquelle R est un groupe acyle linéaire ou ramifié de 2 à 5 atomes de carbone, ou de formule (I') 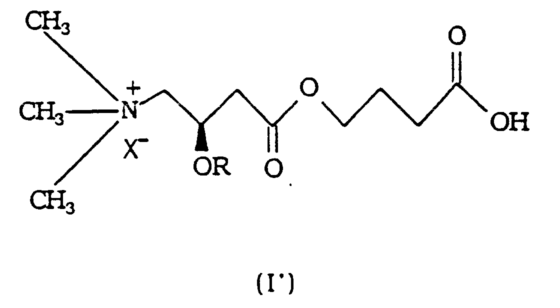 dans laquelle R a la signification indiquée ci-dessus et X⁻ est l'anion d'un acide pharmacologiquement acceptable, pour la préparation d'un médicament pour empêcher les symptômes de sevrage et le besoin d'alcool chez les alcooliques.

2. Utilisation selon la revendication 1 ci-dessus, dans laquelle R est choisi parmi les groupes acétyle, propionyle, n-butyryle, isobutyryle et isovaléryle.

3. Utilisation selon la revendication 1 ou 2 ci-dessus, dans laquelle X⁻est choisi parmi un chlorure; un bromure; un iodure; un aspartate, en particulier un aspartate acide; un citrate, en particulier un citrate acide; un tartrate; un phosphate, en particulier un phosphate acide; un fumarate, en particulier un fumarate acide; un glycérophosphate; un phosphate de glucose; un lactate; un maléate, en particulier un maléate acide; un orotate; un oxalate, en particulier un oxalate acide; un sulfate, en particulier un sulfate acide; un trichloroacétate; un trifluoroacétate et un méthanesulfonate.
